# EUROPEAN PATENT APPLICATION

(11) **EP 1 142 551 A1**
(43) Date of publication of application: **10.10.2001**
(21) Application number: 00971791.9
(22) Date of filing: 06.11.2000
(51) Int. Cl.: A61K 7/02

(54) **TWO-LAYER MAKEUP COSMETIC COMPOSITION**

(30) Priority: 05.11.1999 JP 31597299
(71) Applicant: SHISEIDO COMPANY LIMITED, Chuo-ku, Tokyo 104-8010 (JP)
(72) Inventor: KUROSAWA, Mari, Shiseido Res. Ctr.(Shin-Yokohama), Yokohama-shi, Kanagawa 224-8558 (JP); KATSUYAMA, T., Shiseido Res. Ctr.(Shin-Yokohama), Yokohama-shi, Kanagawa 224-8558 (JP); HINENO, Teruhiko, Shiseido Res.Ctr.(Shin-Yokohama), Yokohama-shi, Kanagawa 224-8558 (JP)
(74) Representative: Henkel, Feiler, Hänzel
(86) International application number: JP0007775
(87) International publication number: WO0134101

(57) **Abstract**

There is provided a two-layer makeup cosmetic composition containing a first layer foundation composition and a second layer finishing composition, each of the foundation composition and the finishing composition being as follows. First layer foundation composition: 1) a composition containing a silicone oil having a viscosity of 15,000,000 cps or less as measured at 25°C and a powder ingredient; 2) a composition containing a siliconated polysaccharide compound, and a silicone oil having a low viscosity and/or a powder ingredient; or 3) a composition containing i) a silicone oil, ii) a specific polyether-modified silicone, iii) water, and iv) hydrophobic powder. Second layer finishing composition: a composition containing powder with minimized regular reflection and diffuse reflection in an amount of 1-100 wt.% on the basis of the entirety of the finishing composition.

## Description

### Technical Field

The present invention generally relates to a makeup cosmetic composition, and particularly to a two-layer makeup cosmetic composition containing a first layer foundation composition and a second layer finishing composition.

### Background Art

Two-layer makeup cosmetic compositions are used not only for purposes of ordinary makeup, but are particularly useful for effectively concealing wrinkles, skin pores, coarse grains, and other blemishes of the skin.

Japanese Patent Application Laid-Open (*kokai*) No. 6-128122 discloses "a multi-layer cosmetic composition for concealing wrinkles, which composition contains a first layer makeup foundation composition including an adhesive substance and a second layer makeup-finishing composition including light-diffuse-reflecting powder" in relation to a multi-layer cosmetic composition for concealing wrinkles (hereinafter the composition may be referred to as the "conventional multi-layer cosmetic composition").

An adhesive substance is included in the first layer composition of the conventional multi-layer cosmetic composition, and thus permits the second layer makeup-finishing composition to be placed onto the first layer composition. Since the second layer composition includes light-diffuse-reflecting powder, the composition exerts the effect of concealing wrinkles, skin pores, coarse grains, and other blemishes of the skin (see the aforementioned publication, page 3, paragraph No. 0024).

However, the conventional multi-layer cosmetic composition involves some problems. The first layer makeup-foundation composition of the conventional multi-layer cosmetic composition is so formulated that would primarily attain the purpose of allowing the second layer composition to be applied reliably onto the skin through the first layer (specifically, the first layer composition includes an adhesive substance such as an acrylic copolymer), and thus the first layer composition is not imparted with a function for concealing skin wrinkles or skin pores. Therefore, when the conventional multi-layer cosmetic composition is used, in most cases, skin pores, etc. are concealed merely by means of the function of "powder" contained in the second layer makeup-finishing composition.

Thus, the primary function to be exerted by the aforementioned conventional multi-layer cosmetic composition is to provide a smooth appearance to skin pores, etc. through diffuse reflection of light applied to the skin pores by means of a light diffuse reflection function of "powder" contained in the second layer makeup-finishing composition. (In general, powder has a "regular reflection function" in addition to a diffuse reflection function. However, when the regular reflection function is predominant over the diffuse reflection function, light falling on skin pores is reflected as it is by the powder, and the skin pores become conspicuous. Therefore, the regular reflection function of powder is not preferable for concealing skin pores, etc.)

However, when diffuse-reflecting powder is present on the surface of the makeup, diffuse-reflected light causes a whitish, powdery appearance; such an appearance is far from a "natural appearance." In this connection, it is to be noted that skin pores are not merely present on the skin, but the periphery of each skin pore is protuberant and the central portion forms a dent. In addition, since each skin pore assumes a reddish or dark tone, the tone of the skin pore differs conspicuously from that of the portion surrounding the skin pore. Therefore, in order to conceal skin pores, etc., a large amount of a highly concealable pigment having an excellent light diffuse reflection function (e.g., titanium oxide) must be incorporated into the second layer composition. Users of the conventional multi-layer cosmetic composition can conceal skin pores, etc., but may suffer from the problem of the surface of the skin exhibiting a powdery appearance.

Recently, it has become important for makeup to have a natural appearance. Therefore, there has been demand for a novel multi-layer makeup cosmetic composition which enables complete concealment of skin pores, etc. and realizes a natural appearance.

However, finding a solution to the aforementioned problems is not easy. This is because, in the case in which powder with minimized light regular reflection and light diffuse reflection is incorporated into the second layer makeup-finishing composition, when the conventional multi-layer cosmetic composition is used, skin pores are no longer concealed effectively by means of the function of the powder; i.e., the original purpose of the multi-layer cosmetic composition cannot be attained.

In view of the foregoing, an object of the present invention is to provide a makeup cosmetic composition which exhibits excellent concealability of skin pores, etc., and realizes a natural appearance, which composition overcomes the aforementioned problems involved in the conventional multi-layer makeup cosmetic composition.

### Disclosure of the invention

In order to solve the aforementioned problems, the present inventors have performed studies on means which can provide a natural appearance and conceal skin pores, etc., even when "powder with minimized regular reflection and diffuse reflection" is used.

As a result, the present inventors have found that, when a specific composition which can adhere to skin pores, etc., and smooth irregularities of the skin pores, etc. is used as a first layer makeup foundation composition, a multi-layer makeup cosmetic composition having the desired effect can be obtained.

Accordingly, the present invention provides a two-layer makeup cosmetic composition (hereinafter the composition may be referred to as "the multi-layer cosmetic composition of the present invention") comprising a first layer foundation composition and a second layer finishing composition, with both the foundation composition and the finishing compositior being as described below.

First layer foundation composition: (1) a composition containing a silicone oil having a viscosity of 15,000,000 cps or less as measured at 25°C and a powder ingredient; (2) a composition containing a siliconated polysaccharide compound, and a silicone oil having a low viscosity and/or a powder ingredient; or (3) a composition containing i) a silicone oil, ii) a polyether-modified silicone represented by the following formula, iii) water, and iv) hydrophobic powder: 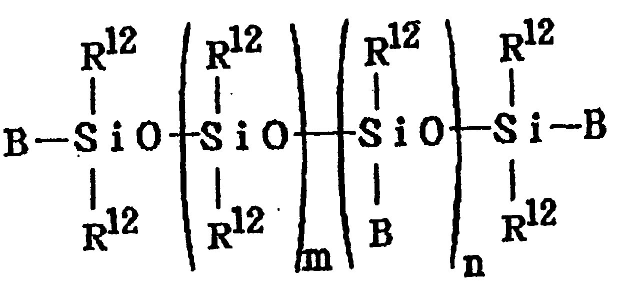 [wherein B represents a methyl group, a phenyl group, or a polyoxyalkylene group represented by the general formula -C₃H₆O(C₂H₄O)_{b}(C₃H₆O)_{c}R¹³ (wherein R¹³ represents a hydrogen atom, an acyl group, or a C1-C4 alkyl group; and each of b and c represents an integer of 5-50); R¹² represents a methyl group or a phenyl group; m represents an integer of 50-1,000; n represents an integer of 1-40; and the molecule contains at least one polyoxyalkylene group represented by the above formula].

Second layer finishing composition: a composition containing powder with minimized regular reflection and diffuse reflection in an amount of 1-100 wt.% on the basis of the entirety of the finishing composition.

### Brief Description of Drawings

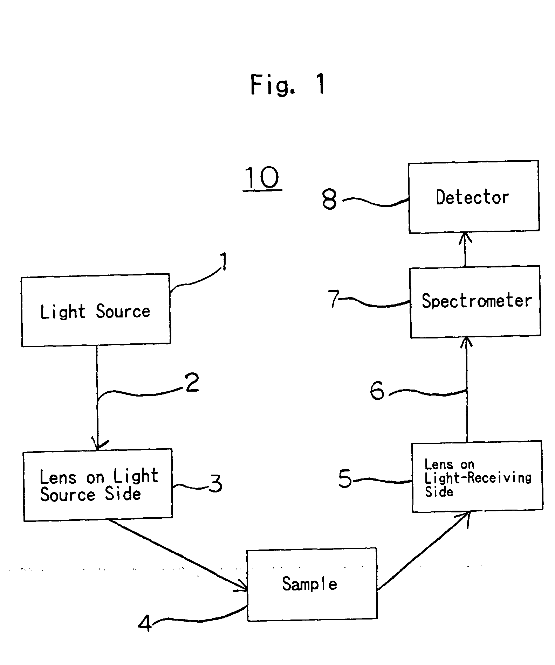

Fig. 1 is a block diagram showing the structure of an actinometer used in the Examples.

### Best Mode for Carrying Out the Invention

Embodiments of the present invention will next be described.

As described above, the multi-layer cosmetic composition of the present invention is composed of (A) a first layer foundation composition and (B) a second layer finishing composition.

### (A) First layer foundation composition

The first layer foundation composition is (1) a composition containing a silicone oil having a viscosity of 15,000,000 cps or less as measured at 25°C and a powder ingredient; (2) a composition containing a siliconated polysaccharide compound, and a silicone oil having a low viscosity and/or a powder ingredient; or (3) a composition containing i) a silicone oil, ii) a polyether-modified silicone represented by the aforementioned formula, iii) water, and iv) hydrophobic powder. Each of the above compositions can adhere to blemishes of the skin, such as skin pores, wrinkles, coarse grains, and scars (as used herein, such blemishes may be referred to as "skin pores, etc." or "skin blemishes"), to thereby smooth the skin pores, etc. Each composition will be described hereunder.

### (1) Composition containing a silicone oil having a viscosity of 15,000,000 cps or less as measured at 25°C and a powder ingredient (hereinafter the composition may be referred to as "the first foundation composition")

The first foundation composition is a composition substantially the same as the "composition for correcting irregularities" disclosed in Japanese Patent Application Laid-Open (*kokai*) No. 11-60445.

The viscosity of a silicone oil which may be incorporated into the first foundation composition is 15,000,000 cps or less. In order to facilitate the application of the first foundation composition to the skin to a possibly greatest extent, the viscosity of the silicone oil is preferably 10,000-1,000,000 cps, more preferably 10,000-100,000 cps.

Specific examples of the silicone oil include organopolysiloxanes such as dimethylpolysiloxane represented by the following formula: 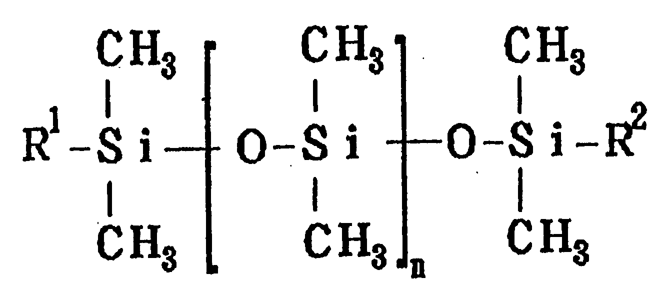 (wherein each of R¹ and R², which may be identical to or different from each other, represents a methyl group or a hydroxyl group; and n represents an average polymerization degree of 500-20,000), methylhydrogenpolysiloxane, methylphenylpolysiloxane, methylcyclopolysiloxane, alkyl-modified silicone, amino-modified silicone, epoxy-modified silicone, carboxyl-modified silicone, chloroalkyl-modified silicone, alkyl-higher-alcohol-ester-modified silicone, alcohol-modified silicone, polyether-modified silicone, and fluorine-modified silicone. Particularly, dimethylpolysiloxane represented by the aforementioned formula, methylhydrogenpolysiloxane, or methylphenylpolysiloxane is preferably incorporated into the first foundation composition, from the viewpoint of assured safety of the resultant first foundation composition during use on the skin, and transparency, and availability at the present time.

The aforementioned silicone oil may be produced through a conventionally known process, or may be a commercially available silicone oil.

These silicone oils may be incorporated into the first foundation composition singly or, if necessary, in combination of two or more species. When a silicone oil mixture prepared through mixing of silicone oils having different viscosity values so as to attain a viscosity falling within a range of 10,000-15,000,000 cps, preferably 10,000-1,000,000 cps, more preferably 10,000-100,000 cps, is incorporated into the first foundation composition, adhesion of the composition to the skin is enhanced, and a sticky sensation during use can be suppressed.

Specifically, when two or more species of a low-viscosity silicone oil having an average viscosity of at least 1 cps and less than 10,000 cps [e.g., SH200C (1-5,000 cps), product of Dow Corning Toray Silicone Co., Ltd.], a middle-viscosity silicone oil having an average viscosity of at least 10,000 cps and less than 1,000,000 cps [e.g., SH200 (10,000-1,000,000 cps), product of Dow Corning Toray Silicone Co., Ltd.], and a high-viscosity silicone oil having an average viscosity of 1,000,000-15,000,000 cps are incorporated into the first foundation composition, to thereby attain a desired viscosity, the aforementioned "enhancement of adhesion and suppression of a sticky sensation in use" can be attained.

No particular limitation is imposed on the amount of the silicone oil incorporated into the first foundation composition, since the amount of the silicone oil is appropriately determined in accordance with the intended characteristics of the composition, and the amount of the silicone oil varies with the type of the silicone oil employed. In general, the amount of the silicone oil is preferably 80.0 wt.% or more on the basis of the entirety of the composition exclusive of powder ingredient.

When the amount of the silicone oil incorporated into the first foundation composition is below the above range, the foundation composition becomes opaque, and thus the presence of the composition tends to be apparent when the composition is applied onto the skin.

When a hydrocarbon polymer is incorporated into the first foundation composition instead of the silicone oil, adhesion of the resultant composition becomes excessively high, and thus removal of the composition applied onto the skin tends to become very difficult. Therefore, such a hydrocarbon polymer is not preferable as an ingredient incorporated into the foundation composition.

The first foundation composition contains a powder ingredient together with the silicone oil.

When a powder ingredient is incorporated into the foundation composition, running of the composition, which is attributed to the silicone oil (which is a Newtonian fluid), can be prevented, and the degree of correction to irregularities on the skin can further be enhanced.

A powder ingredient which may be incorporated into the first foundation composition is not particularly limited. Examples of the powder ingredient include inorganic powders such as talc, kaolin, mica, sericite, muscovite, phlogopite, synthetic mica, lepidolite, biotite, lithia mica, vermiculite, magnesium carbonate, calcium carbonate, aluminum silicate, barium silicate, calcium silicate, magnesium silicate, strontium silicate, metal tungstates, magnesium, silicon dioxide, zeolite, barium sulfate, calcined calcium sulfate (calcined gypsum), calcium phosphate, fluorine apatite, hydroxyapatite, ceramic powder, metallic soap (zinc myristate, calcium palmitate, aluminum stearate), and boron nitride; and organic powders such as polyamide resin powder (nylon powder), polyethylene powder, polymethyl methacrylate powder, polystyrene powder, styrene-acrylic acid copolymer resin powder, benzoguanamine resin powder, silicone resin powder, silicone rubber powder, polytetrafluoroethylene powder, and cellulose powder. If necessary, the powder ingredient may be coated with a silicone compound, a fluorine-modified silicone compound, a fluorine compound, a higher fatty acid, a higher alcohol, a fatty acid ester, metallic soap, an amino acid, or an alkyl phosphate, and incorporated into the first foundation composition.

Of the aforementioned powder ingredients, a powder ingredient having a refractive index of about 1.3-1.5 is preferably incorporated into the first foundation composition. Refractive indices falling within the above range almost overlap the refractive index range of the above-listed silicone oils, any of which serves as another incorporation ingredient. Therefore, when such a powder ingredient and the silicone oil are incorporated in combination, transparency of the first foundation composition, which is attributed to the silicone oil, is not impaired.

Specific examples of the powder ingredient having a refractive index falling within the above range include silicon dioxide powder, silicone resin powder, and silicone rubber powder.

Silicon dioxide powder is commercially available as silica powder [e.g., Chemiceren (product of Sumitomo Chemical Co., Ltd.), Spherical Silica P-1500 (product of Catalysts and Chemicals Industries Co., Ltd.), Aerosil #200 (product of Deggusa), Aerosil R972, and Sildex L-51 (product of Asahi Glass Co., Ltd.)]. In the present invention, such a commercially available powder may be used. When such silicon dioxide powder is incorporated into the first foundation composition, adhesion of the first foundation composition to the skin can be enhanced.

Silicone resin powder is obtained by pulverizing silicone resin produced through copolymerization of multifunctional siloxane ingredients. A variety of silicone resin powders are commercially available. Examples of such commercially available silicone resin powder include Tospearl (e.g., Tospearl 145A) produced by Toshiba Silicone Co., Ltd.

Since such silicone resin powder has a refractive index almost the same as that of the silicone oil, use of such silicone resin powder and the silicone oil in combination can provide a transparent system.

Silicone rubber powder is organopolysiloxane powder produced from an organopolysiloxane elastomer composition or an organopolysiloxane resin composition. A variety of silicone rubber powders are commercially available. Examples of such commercially available silicone rubber powders include Trefil (e.g., Trefil E505C, Trefil E506C, or Trefil E505W) produced by Dow Corning Toray Silicone Co., Ltd.

Such silicone rubber powder has high elasticity as compared with other powder ingredients. Therefore, when such silicone rubber powder is incorporated into the first foundation composition, the elasticity of the composition can be approximated to that of the skin, to thereby produce a state similar to that of the real skin.

In addition, when a powder ingredient having an almost spherical shape is incorporated into the first foundation composition, the composition is easily applied onto the skin. Therefore, spherical powder of the aforementioned silicon dioxide powder, silicone resin powder, and/or silicone rubber powder (e.g., the aforementioned Tospearl produced by Toshiba Silicone Co., Ltd.) is preferably incorporated into the first foundation composition.

The aforementioned powder ingredients may be incorporated into the first foundation composition singly or, if necessary, in combination of two or more species. For example, when silicone rubber powder and silicon dioxide micropowder are incorporated in combination into the first foundation composition, the composition is easily applied onto the skin, and adhesion of the composition to the skin can be enhanced.

In order to secure the transparency of the first foundation composition, a powder ingredient having the aforementioned refractive index (1.3-1.5) preferably accounts for 90 wt.% or more of the entirety of the powder ingredient incorporated into the composition. When a powder ingredient having a refractive index falling outside the above range accounts for more than 10 wt.% of the entirety of the powder ingredient incorporated into the first foundation composition, the composition becomes colored, and the transparency of the composition tends to be lowered.

No particular limitation is imposed on the amount of the powder ingredient incorporated into the first foundation composition, since the amount of the powder ingredient should be appropriately determined in accordance with the type of the powder ingredient or the specific surface area thereof (the larger the specific surface area of the powder ingredient, the less the preferable amount of the powder ingredient). In general, the amount of the powder ingredient is preferably 10.0-80.0 wt.% on the basis of the entirety of the composition.

When the amount of the powder ingredient is less than 10.0 wt.% on the basis of the entirety of the first foundation composition, running of the composition when the composition is applied onto the skin is not satisfactorily prevented, whereas when the amount of the powder ingredient is in excess of 80.0 wt.% on the basis of the entirety of the composition, the composition tends to encounter difficulty in assuming a paste-like state, which is not preferable.

As described above, when the silicone oil having a viscosity of 15,000,000 cps or less and the powder ingredient are incorporated in combination into the first foundation composition, the composition exhibits the desired characteristics.

### (2) Composition containing a siliconated polysaccharide compound, and a silicone oil having a low viscosity and/or a powder ingredient (hereinafter the composition may be referred to as "the second foundation composition")

A siliconated polysaccharide compound contained in the second foundation composition is disclosed in Japanese Patent Application Laid-Open (*kokai*) No. 10-29910, and is specifically represented by the following formula (I): 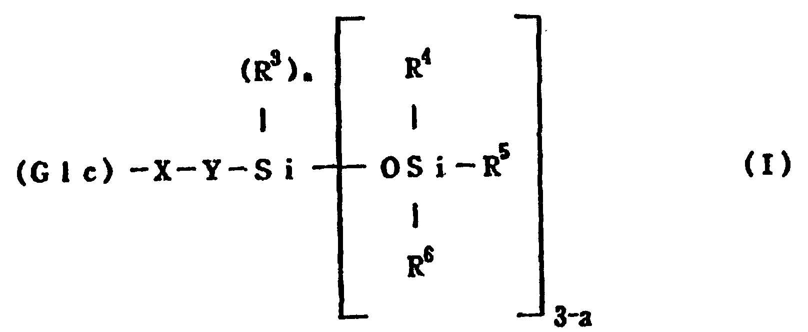 [wherein G1c represents a sugar residue of a polysaccharide compound; X represents a divalent bond group; Y represents a divalent aliphatic group; R³ represents a C1-C8 monovalent organic group; each of R⁴, R⁵, and R⁶ represents a C1-C8 monovalent organic group or a siloxy group represented by-OSiR⁷R⁸R⁹ (wherein each of R⁷, R⁸, and R⁹ represents a C1-C8 monovalent organic group); and a denotes 0, 1, or 2].

In formula (I), G1c represents a sugar residue of a polysaccharide compound. Examples of the polysaccharide compound include a variety of known polysaccharides such as cellulose, hemicellulose, gum arabic, tragacanth gum, tamarind gum, pectin, starch, mannan, guar gum, locust bean gum, quince seed gum, alginic acid, carrageenan, agar, xanthane gum, dextran, pullulan, chitin, chitosan, hyaluronic acid, and chondroitin sulfuric acid; and polysaccharide derivatives such as carboxymethylated derivatives, sulfated derivatives, phosphated derivatives, methylated derivatives, ethylated derivatives, derivatives obtained through addition of alkylene oxide such as ethylene oxide or propylene oxide, acylated derivatives, cationized derivatives, and low molecular weight derivatives. Of these polysaccharide compounds, ethyl cellulose or pullulan is preferably chosen. More preferably, pullulane is chosen. The molecular weight of the polysaccharide compound, although varies with the type of compound, is about 1,000-5,000,000.

Such a polysaccharide compound contains at least one of a plurality of species of a reactive functional group such as a hydroxyl group and a carboxyl group. The divalent bond group represented by X is formed through reaction between a reactive functional group contained in such a polysaccharide compound and a silicone compound represented by the following formula (II): 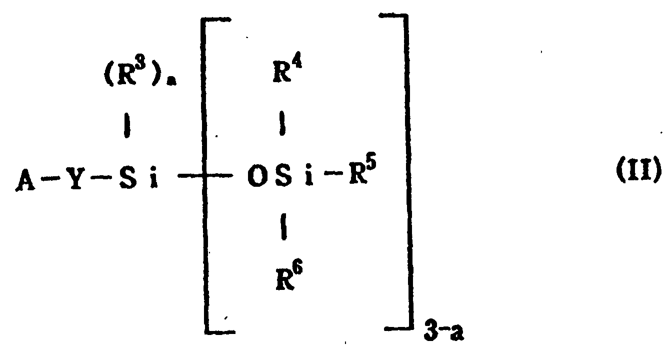 [wherein Y, R³, R⁴, R⁵, R⁶, and a have the same meanings as in formula (I); and A represents a functional group which can be reacted with the reactive functional group contained in the polysaccharide compound, such as an isocyanate group, an epoxy group, a vinyl group, an acryloyl group, a methacryloyl group, an amino group, an imino group, a hydroxyl group, a carboxyl group, or a mercapto group]. The divalent bond group represented by X is derived from the functional group A. Reaction between such a polysaccharide compound and such a silicone compound may be carried out by means of a conventionally known method.

Examples of the divalent bond group X formed as described above include a carbamoyl group, -CH₂CH(OH)-, a carbonyl group, an amino group, and an ether group. In consideration of reactivity, a carbamoyl group (-CONH-) is preferable, the carbamoyl group being formed through reaction between a compound represented by formula (II) in which A is an isocyanate group (O=C=N-) and a hydroxyl group (i.e., a reactive functional group) of the polysaccharide compound. In this case, the sugar residue G1c refers to a residual portion of the polysaccharide compound exclusive of a hydrogen atom of the hydroxyl group reacted with the isocyanate group. In the case in which the divalent bond group X is formed through a different reaction, the term "the sugar residue G1c" is to be understood to mean likewise.

Y represents a divalent aliphatic group. Examples of the divalent aliphatic group include an alkylene group; an alkylene group containing an oxygen atom, a nitrogen atom, or a sulfur atom in the main chain; an alkylene group containing an arylene group such as a phenylene group in the main chain; and an alkylene group containing a carbonyloxy group or an oxycarbonyl group in the main chain. The divalent aliphatic group may contain a substituent such as a hydroxy group, an alkoxy group, or an alkyl group. A terminal atom of the aliphatic group may be a hetero atom such as an oxygen atom, a nitrogen atom, or a sulfur atom. Examples of the divalent aliphatic group Y include - (CH₂)₂-, -(CH₂)₃-, -(CH₂)₄-, -(CH₂)₆-, - (CH₂)₈-, -[CH₂CH(CH₃)]-, -(CH₂)₂(CH₂)₃, and -CH₂CH(OH)-CH₂-. Of these, a propylene group represented by -(CH₂)₃- is preferable.

Each of R³, R⁷, R⁸, and R⁹ represents a C1-C8 monovalent organic group, and each of R⁴, R⁵, and R⁶ may represent a C1-C8 monovalent organic group. Examples of such a monovalent organic group include alkyl groups such as a methyl group, an ethyl group, a propyl group, and a butyl group; cycloalkyl groups such as a cyclopentyl group and a cyclohexyl group; aryl groups such as a phenyl group; aralkyl groups such as a benzyl group; alkenyl groups such as a vinyl group and an allyl group; and fluorinated alkyl groups such as a 3,3,3-trifluoropropyl group.

Each of R⁴, R⁵, and R⁶ may represent a siloxy group represented by -OSiR⁷R⁸R⁹. Examples of such a siloxy group include a trimethylsiloxy group, an ethyldimethylsiloxy group, a phenyldimethylsiloxy group, a vinyldimethylsiloxy group, and a 3,3,3-trifluoropropyldimethylsiloxy group.

R³, R⁴, R⁵, R⁶, R⁷, R⁸, and R⁹ may be identical to or different from one another, but each of R⁴, R⁵, and R⁶ is preferably a methyl group.

In formulas (I) and (II), a denotes 0, 1, or 2, and is preferably 0.

A particularly preferable siliconated polysaccharide compound used in the second foundation composition is siliconated pullulan (Japanese Patent Application Laid-Open (*kokai*) No. 3-134103) represented by the following formula (III): 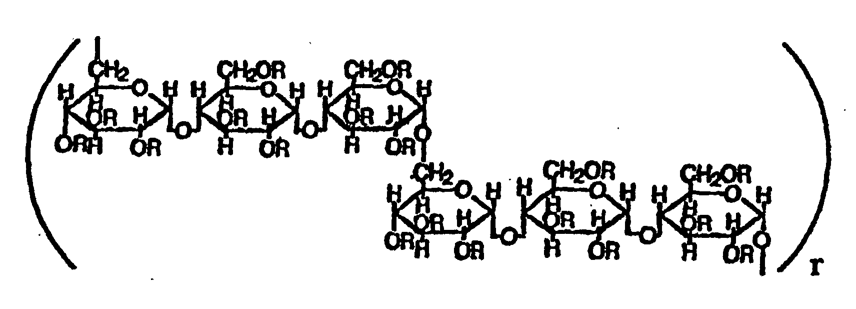 [wherein R represents a hydrogen atom or a [(CH₃)₃SiO]₃Si(CH₃)₃NHCO group; and r represents a positive number].

In the siliconated polysaccharide compound incorporated into the second foundation composition, the bonding percentage of a silicone compound with respect to a reactive functional group of a polysaccharide compound may fail to be 100%. However, when the bonding percentage of a silicone compound with respect to a polysaccharide compound is very low, the effects of the present invention are not satisfactorily obtained, which is not preferable. The bonding percentage varies with the type of silicone compound and polysaccharide compound. The average bonding number (substitution degree) of a silicone compound with respect to a sugar unit constituting a polysaccharide compound is preferably 0.5-2.5. The substitution degree is calculated on the basis of the amount (wt.%) of Si contained in the siliconated polysaccharide compound.

A siliconated polysaccharide compound produced through a conventionally known process may be incorporated into the second foundation composition. Alternatively, a commercially available siliconated polysaccharide compound may be incorporated into the composition.

No particular limitation is imposed on the amount of the siliconated polysaccharide compound incorporated into the second foundation composition, since the amount of the polysaccharide compound is appropriately determined in accordance with the intended characteristics of the composition, and the amount of the polysaccharide compound varies with the type of compound. In general, the amount of the polysaccharide compound is preferably 0.1-40.0 wt.% on the basis of the entirety of the composition. In the case in which the amount of the siliconated polysaccharide compound is less than 0.1 wt.% on the basis of the entirety of the composition, when the composition is applied onto the skin, the thickness of the resultant composition layer is not satisfactory for cocealing irregularities on the skin. In addition, the degree of adhesion of the composition to the skin and cohesion of the composition are not satisfactory, which is not preferable. In contrast, when the amount of the siliconated polysaccharide compound is in excess of 40.0 wt.% on the basis of the entirety of the composition, the composition cannot be spread over the skin, which is not preferable.

Preferably, the second foundation composition contains, in addition to the aforementioned siliconated polysaccharide compound, a silicone oil having a low viscosity.

Examples of such a low-viscosity silicone oil include a low molecular weight silicone oil represented by the following formula (IV): 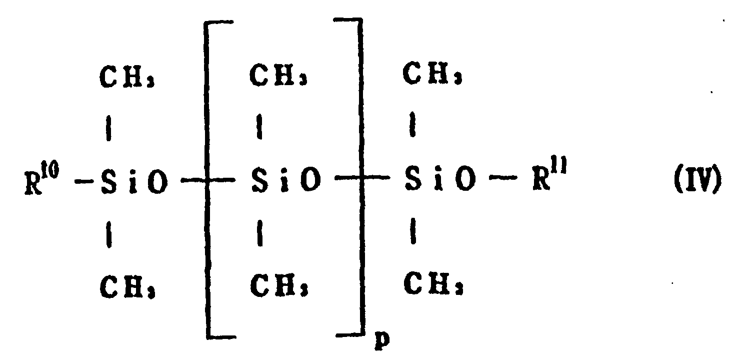 (wherein each of R¹⁰ and R¹¹, which may be identical to or different from each other, represents a methyl group or a hydroxyl group; and p represents an integer of 0-5); or the following formula (V): 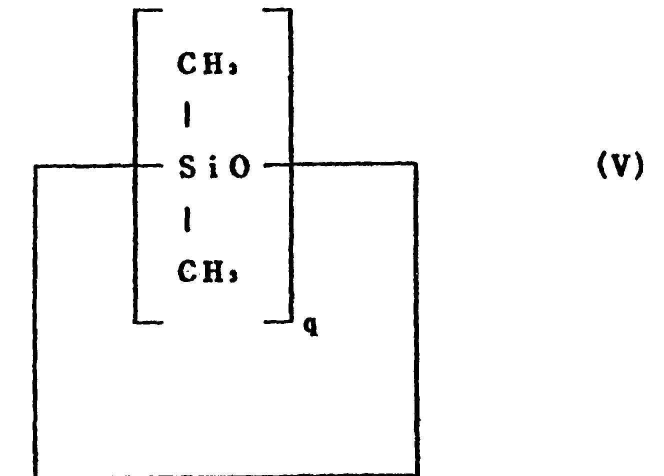 (wherein q denotes an integer of 3-7). An example of such a low molecular weight silicone oil is dimethylpolysiloxane, which is represented by formula (IV) in which each of R¹⁰ and R¹¹ is a methyl group.

Furthermore, examples of such a silicone oil include organopolysiloxanes such as methylhydrogenpolysiloxane, methylphenylpolysiloxane, methylcyclopolysiloxane, alkyl-modified silicone, amino-modified silicone, epoxy-modified silicone, carboxyl-modified silicone, chloroalkyl-modified silicone, alkyl-higher-alcohol-ester-modified silicone, alcohol-modified silicone, polyether-modified silicone, and fluorine-modified silicone.

Of these silicone oils, dimethylpolysiloxane, methylhydrogenpolysiloxane, or methylphenylpolysiloxane is preferably incorporated into the second foundation composition, from the viewpoints of safety of the resultant second foundation composition against the skin, transparency, and availability at the present time.

The low-viscosity silicone oil which may be incorporated into the second foundation composition preferably has a viscosity of 1-1,000 mPa·s at 25°C, more preferably 1-100 mPa·s at 25°C.

The low-viscosity silicone oil incorporated into the second foundation composition may be produced through a conventionally known process, or may be a commercially available low-viscosity silicone oil.

Preferably, the second foundation composition contains, in addition to the aforementioned siliconated polysaccharide compound, one or more powder ingredients.

A powder ingredient which may be incorporated into the second foundation composition is not particularly limited. Examples of the powder ingredient include inorganic powder ingredients such as talc, kaolin, mica, sericite, muscovite, phlogopite, synthetic mica, lepidolite, biotite, lithia mica, vermiculite, magnesium carbonate, calcium carbonate, aluminum silicate, barium silicate, calcium silicate, magnesium silicate, strontium silicate, metal tungstates, magnesium, silicon dioxide, zeolite, barium sulfate, calcined calcium sulfate (calcined gypsum), calcium phosphate, fluorine apatite, hydroxyapatite, ceramic powder, metallic soap (zinc myristate, calcium palmitate, aluminum stearate), and boron nitride; and organic powder ingredients such as polyamide resin powder (nylon powder), polyethylene powder, polymethyl methacrylate powder, polystyrene powder, styrene-acrylic acid copolymer resin powder, benzoguanamine resin powder, silicone resin powder, silicone rubber powder, silicone resin-coated rubber powder, polytetrafluoroethylene powder, and cellulose powder.

If necessary, the powder ingredient may be surface-treated with a silicone compound, a fluorine-modified silicone compound, a fluorine compound, a higher fatty acid, a higher alcohol, a fatty acid ester, metallic soap, an amino acid, or an alkyl phosphate, and then incorporated into the second foundation composition.

Of the aforementioned powder ingredients, a powder ingredient having a refractive index (as used herein, refractive index refers to a refractive index calculated according to Snell's law) of about 1.3-1.5 is preferably incorporated into the second foundation composition as the whole or a portion of the powder ingredient. A refractive index falling within the above range is almost the same as the refractive index of the siliconated polysaccharide compound or the low-viscosity silicone oil serving as an incorporation ingredient. Therefore, when such a powder ingredient having a refractive index falling within the above range is incorporated in combination with the siliconated polysaccharide compound and the low-viscosity silicone oil, transparency of the siliconated polysaccharide compound and the low-viscosity silicone oil is not impaired within a desired range in the resultant second foundation composition.

Specific examples of a powder ingredient having a refractive index falling within the above range include silicon dioxide powder, silicone resin powder, silicone rubber powder, and silicone resin-coated rubber powder.

Silicon dioxide powder is commercially available as silica powder [e.g., Chemiceren (product of Sumitomo Chemical Co., Ltd.), Spherical Silica P-1500 (product of Catalysts and Chemicals Industries Co., Ltd.), Aerosil #200 (product of Deggusa), Aerosil R972, and Sildex L-51 (product of Asahi Glass Co., Ltd.)]. In the second foundation composition, such a commercially available powder may be used. When such silicon dioxide powder is incorporated into the second foundation composition, adhesion of the composition to the skin can be enhanced.

Silicone resin powder is obtained by pulverizing silicone resin produced through copolymerization of multifunctional siloxane ingredients. A variety of silicone resin powders are commercially available. Examples of such commercially available silicone resin powder include Tospearl (e.g., Tospearl 145A) produced by Toshiba Silicone Co., Ltd. Such silicone resin powder has a refractive index almost identical to that of the siliconated polysaccharide compound and the low-viscosity silicone oil. Therefore, when such silicone resin powder is incorporated in combination with the siliconated polysaccharide compound and the low-viscosity silicone oil, transparency of the second foundation composition can be secured.

Silicone rubber powder is organopolysiloxane powder produced from an organopolysiloxane elastomer composition or an organopolysiloxane resin composition. Examples of such silicon rubber powder include hardened organopolysiloxane powder having a particle size of 100 microns or less (Japanese Patent Publication (*kokoku*) No. 4-17162). A variety of silicone rubber powders are commercially available. Examples of such commercially available silicone rubber powder include Trefil (e.g., Trefil E506W, Trefil E505C, Trefil E506C, or Trefil E505W) produced by Dow Corning Toray Silicone Co., Ltd. These silicone rubber powders possess high elasticity as compared with other powder ingredients. Therefore, when such silicone rubber powder is incorporated into the second foundation composition, the elasticity of the composition can be made to approximate that of the skin, to thereby cause the state of the composition to be similar to that of real skin.

Silicone resin-coated rubber powder is obtained by coating silicone rubber spherical micropowder with polyorganosilsesquioxane resin (Japanese Patent Application Laid-Open (*kokai*) No. 7-196815). Examples of commercially available silicone resin-coated rubber powder include X-52-1139K produced by Shin-Etsu Chemical Co., Ltd. When such silicone resin-coated rubber powder is incorporated into the second foundation composition, spreadability of the composition on the skin can be enhanced.

A specific type or combination of the powder ingredient which may be incorporated into the second foundation composition can be appropriately determined.

For example, the second foundation composition can be imparted with flexibility when containing, as at least a portion of the aforementioned powder ingredient, a rubber powder ingredient formed of powder particles having an average rubber hardness of less than 50 [as used herein, the term "average rubber hardness" refers to the score obtained through the testing method for durometer hardness (type A) (middle hardness test) according to the hardness test for vulcanized rubber and thermoplastic rubber (JIS K6253)] (typical examples of the rubber powder ingredient include the aforementioned silicone rubber powder and silicone resin-coated rubber powder). When only a rubber powder ingredient formed of powder particles having an average rubber hardness of 50 or more is incorporated into the second foundation composition, the resultant composition becomes very hard, and thus the composition is difficult to spread over the skin, which is not preferable.

When the second foundation composition contains a rubber powder ingredient formed of powder particles having an average rubber hardness of less than 50 in combination with another powder ingredient (e.g., a rubber powder ingredient formed of powder particles having an average rubber hardness of 50 or more or inorganic powder such as silicon dioxide powder), the second foundation composition can exert a long-lasting cosmetic effect (i.e., effective covering of skin grains which may otherwise stand out with passage of time).

In many cases, the particles of a powder ingredient assume a spherical shape or a plate-like shape, or are amorphous. The second foundation composition preferably contains two or more powder ingredients formed of powder particles having different shapes. For example, the composition preferably contains a powder ingredient formed of spherical powder particles (e.g., a rubber powder ingredient) in combination with a powder ingredient formed of amorphous powder particles (e.g., an inorganic powder ingredient such as silicon dioxide powder formed of amorphous particles).

As described above, when two or more powder ingredients formed of powder particles having different shapes, in particular, when a rubber powder ingredient formed of spherical powder particles having an average rubber hardness of less than 50 and an inorganic powder ingredient formed of amorphous powder particles, are incorporated in combination, the long-lasting cosmetic effect (i.e., effective covering of skin grains which may otherwise stand out with passage of time) of the multi-layer cosmetic composition of the present invention can be enhanced, which is preferable.

When a powder ingredient formed of amorphous powder particles is incorporated into the second foundation composition, the thixotropy of the composition can be enhanced, and the stability of the composition with passage of time can be improved. In order to effectively regulate the thickness of the second foundation composition applied onto the skin (particularly in the case in which the composition is used as a "makeup composition"), and to furthermore enhance the stability of the composition with passage of time, a powder ingredient formed of amorphous powder particles is incorporated into the composition.

When powder ingredients formed of powder particles having different shapes are incorporated in combination into the second foundation composition, the ratio between a powder ingredient formed of spherical particles (particularly a rubber powder ingredient formed of spherical particles) and a powder ingredient formed of particles having a non-spherical shape such as a plate-like shape or formed of amorphous particles may be appropriately determined in accordance with the specific form or the purpose of the second foundation composition. Therefore, the ratio is not particularly limited.

The majority of the entirety of powder ingredients incorporated into the second foundation composition may be a powder ingredient formed of powder particles having any one of the aforementioned shapes (inclusive of a powder ingredient formed of amorphous powder particles). Alternatively, the amount of a powder ingredient formed of spherical particles may be equal to that of a powder ingredient formed of non-spherical particles.

For example, when a powder ingredient formed of amorphous particles is incorporated into the second foundation composition in combination with a powder ingredient formed of particles having another shape, the amount of the powder ingredient formed of amorphous particles usually falls within a very wide range; i.e., about 10-90 wt.% on the basis of the entirety of the powder ingredients incorporated into the composition (the above range is a yardstick).

In the case in which the amount of the powder ingredient formed of amorphous particles is relatively small (for example, less than about 10 wt.% on the basis of the entirety of the powder ingredients), (1) when the second foundation composition is intended to be used for correcting large irregularities on the skin (e.g., sears or scars) by smoothing them out, long-lasting cosmetic effect (effective covering of skin grains which may otherwise stand out with passage of time) is not satisfactory; and (2) when the second foundation composition is intended to be used for correcting relatively small irregularities on the skin (e.g., skin pores or wrinkles) by smoothing them out, the composition fails to exert a satisfactorily effect. In contrast, in the case in which the amount of the powder ingredient formed of amorphous particles is relatively large (for example, in excess of about 90 wt.% on the basis of the entirety of the powder ingredients), (1) when the second foundation composition is intended to be used for smooth-correcting large irregularities on the skin (e.g., sears or scars), the composition fails to exhibit satisfactory flexibility and cohesion, and thus when the composition is applied onto the skin, the composition easily breaks down into fragments as a result of skin movement; and (2) when the second foundation composition is intended to be used for smooth-correcting relatively small irregularities on the skin (e.g., skin pores or wrinkles), the stability of the composition with passage of time tends to lower.

No particular limitation, is imposed on the amount of the powder ingredient incorporated into the second foundation composition, since the amount of the powder ingredient should be appropriately determined in accordance with the type of the powder ingredient or the specific surface area thereof (the larger the specific surface area of the powder ingredient, the less the preferable amount of the powder ingredient). In general, the amount of the powder ingredient is preferably 5.0-80.0 wt.% on the basis of the entirety of the composition. When the amount of the powder ingredient is less than 5.0 wt.% on the basis of the entirety of the second foundation composition, running of the composition when the composition is applied onto the skin is not satisfactorily prevented, whereas when the amount of the powder ingredient is in excess of 80.0 wt.% on the basis of the entirety of the composition, the composition tends to encounter difficulty in even assuming a paste-like state.

If necessary, the second foundation composition may contain a volatile ingredient in addition to the aforementioned ingredients. The volatile ingredient may be any of the volatile ingredients which are widely used in compositions for external use, such as cosmetic compositions. Specific examples of the volatile ingredient include volatile silicone oils, water, lower alcohols, and mixtures thereof. Such a volatile ingredient may be appropriately chosen in accordance with the specific embodiment or the product form (e.g., oil or emulsion) of the second foundation composition. When such a volatile ingredient is incorporated into the second foundation composition, the viscosity of the composition can be regulated as a product so as to be suitable for use, and the thickness of the composition applied onto the skin can be regulated.

The volatile silicone oil may be any of the volatile silicone oils which are used in compositions for external use, such as cosmetic compositions, and is not particularly limited. Specific examples of the silicone oil include low-boiling-point chain silicone oils such as hexamethyldisiloxane, and octamethyltrisiloxane; and low-boiling-point cyclic silicone oils such as octamethylcyclotetrasiloxane, decamethylcyclopentasiloxane, dodecamethylcyclohexasiloxane, tetradecamethylcycloheptasiloxane.

Examples of the lower alcohol include ethanol.

### Specific embodiments of the second foundation composition

As described above, embodiments of the second foundation composition include (1) a composition for correction of large irregularities on the skin (e.g., sears or scars) by smoothing them out (i.e., for correction of irregularities); and (2) a composition for correcting relatively small irregularities on the skin (e.g., skin pores or wrinkles) by smoothing them out (i.e., for makeup).

### The case in which the second foundation composition is used for correction of irregularities

In this case, the purpose of the composition is to effectively correct relatively large irregularities on the skin, including (1) conspicuous irregularities on the skin such as crater-like irregularities due to acne, (2) keloid scars or skin graft scars due to burning, (3) surgical scars, (4) deep wrinkles, and (5) deep scars.

In order to attain the aforementioned purpose effectively, the composition is usually required to exhibit characteristics such that the composition can be applied onto the skin relatively thickly (i.e., about 1-3 mm) and to exhibit excellent transparency.

In order to realize "thick application of the composition onto the skin" and "transparency of the composition," it is preferable that the second foundation composition satisfies the below-described conditions.

No particular limitation is imposed on the amount of a low-viscosity silicone oil incorporated into the second foundation composition. When the composition for correction of irregularities of the present invention contains a low-viscosity silicone oil in combination with a siliconated polysaccharide compound, so as to attain a viscosity of 10,000-15,000,000 mPa·s at 25°C, preferably 10,000-1,000,000 mPa·s at 25°C, more preferably 10,000-100,000 mPa·s at 25°C, the second foundation composition can be applied onto the skin thickly, and adhesion of the composition to the skin and the spreadability of the composition over the skin can be enhanced [as used herein, unless otherwise specified, the term "viscosity" refers to a viscosity as measured by use of Shibaura Vismetron (product of Shibaura System) in a thermostatic chamber at 25°C under the following conditions: rotor No. 7, revolution number 0.5 rpm].

The total amount of a siliconated polysaccharide compound and a silicone oil incorporated into the second foundation composition is preferably 70.0 wt.% or more on the basis of the entirety of the composition exclusive of the aforementioned powder ingredient (or exclusive of the powder ingredient and a volatile ingredient when the volatile ingredient is incorporated into the second foundation composition). When the total amount of the siliconated polysaccharide compound and the silicone oil falls within the above range, the composition exhibits excellent transparency. Therefore, the composition can be more uniformly applied onto the skin such that irregularities on the skin are not conspicuous regardless of the size and the depth of the irregularities.

A powder ingredient having a refractive index of 1.3-1.5 preferably accounts for 90 wt.% or more of the entirety of the powder ingredient incorporated into the second foundation composition, in order to maintain the transparency of the second foundation composition [specifically, in order to maintain the transparency at ΔL of 20 or more, wherein "ΔL" is the difference between the Hunter Lab L value of the color measured by use of a white portion of a concealment percentage test sheet as a background (white back) and the Hunter Lab L value of the color measured by use of a black portion of the test sheet as a background (black back), the L value being measured by means of CM-1000 (product of Minolta), (a test composition is applied onto the test sheet so as to attain a thickness of 2 mm)]. When a powder ingredient having a refractive index falling outside the above range accounts for more than 10 wt.% of the entirety of the powder ingredient incorporated into the second foundation composition, the composition becomes colored, and the transparency of the composition tends to be lowered.

### The case in which the second foundation composition is used for makeup

In this case, the composition is used for usual makeup.

In this case, a characteristic feature of the second foundation composition resides in that the composition can naturally conceal large skin pores (e.g., skin pores at pockmarks such as acne; i.e., skin pores having a depth of about 150 µm) and small wrinkles without causing an unfavorable appearance, such large skin pores being difficult to conceal naturally by use of conventional makeup composition (e.g., makeup cosmetic compositions).

In order to effectively conceal such skin pores, the composition is usually required to exhibit characteristics such that the thickness of the composition applied onto the skin can be effectively regulated (specifically the thickness is averaged at about 20 µm) and to exhibit characteristics such that the composition can naturally conceal relatively large skin pores and small wrinkles so as to create the impression that such skin pores and wrinkles do not exist or have never existed.

In order to realize "regulation of the thickness of the composition applied onto the skin" and "natural concealment of large skin pores and small wrinkles," it is preferable the second foundation composition satisfies the below-described conditions.

In the case in which the second foundation composition is used as a makeup composition, when the composition contains a low-viscosity silicone oil in combination with a siliconated polysaccharide compound, so as to attain a viscosity of 10,000-10,000,000 mPa·s at 25°C, preferably 10,000-100,000 mPa·s at 25°C, and further contains a volatile ingredient as an essential ingredient, the thickness of the second foundation composition applied onto the skin can be regulated [the thickness can be regulated to about 20 µm (which is thinner than the case of the composition for correction of irregularities)], and the adhesion of the composition to the skin and the spreadability of the composition over the skin can be enhanced.

When a volatile ingredient is incorporated into the second foundation composition, the composition can be applied onto the skin easily and thinly. After application of the composition, the volatile ingredient contained in the composition vaporizes, and a relatively cohesive composition film is rapidly formed on the skin.

In the case in which the second foundation composition is used for makeup, the total amount of the siliconated polysaccharide compound and the low-viscosity silicone oil incorporated into the composition is preferably 60.0 wt.% or more on the basis of the entirety of the composition exclusive of the aforementioned powder ingredient and the volatile ingredient, since the thickness of the composition applied onto the skin can be regulated to a desired thickness.

In the case in which the second foundation composition is used for makeup, a powder ingredient having a refractive index of 1.3-1.5 preferably accounts for 20 wt.% or more of the entirety of the powder ingredient incorporated into the second foundation composition. In the case in which a powder ingredient having a refractive index of 1.3-1.5 accounts for less than 20.0 wt.% of the entirety of the powder ingredient incorporated into the composition, when the composition is used for concealing large skin pores and small wrinkles, attainment of a natural appearance becomes difficult.

### (3) Composition containing i) a silicone oil, ii) a polyether-modified silicone, iii) water, and iv) hydrophobic powder (hereinafter the composition may be referred to as "the third foundation composition"

The third foundation composition is a composition substantially the same as the "gel cosmetic composition" disclosed in Japanese Patent Application Laid-Open (*kokai*) No. 9-194323.

i) A silicone oil incorporated into the third foundation composition is not particularly limited. Specific examples of the silicone oil include diorganopolysiloxanes having a low viscosity to a high viscosity, such as dimethylpolysiloxane, methylphenylpolysiloxane, and dimethylsiloxane methylphenylsiloxane copolymers; cyclic polysiloxanes such as octamethylcyclotetrasiloxane, decamethylcyclopentasiloxane, and tetramethyltetraphenylcyclotetrasiloxane; gum-like dimethylpolysiloxane having a high polymerization degree, gum-like dimethylsiloxane·methylphenylsiloxane copolymers, or cyclic siloxane solutions of gum-like dimethylpolysiloxane; trimethylsiloxysilicate; cyclic siloxane solutions of trimethylsiloxysilicate; diorganopolysiloxanes having a C6-C50 alkyl group; amino-modified silicones; higher-alkoxy-modified silicones; higher-fatty-acid-modified silicones; alkyl-modified silicones; and fluorine-modified silicones. When, among the aforementioned silicone oils, a cyclic polysiloxane, particularly a cyclic dimethylpolysiloxane, is incorporated into the third foundation composition, the stability of the composition is enhanced.

No particular limitation is imposed on the amount of the silicone oil incorporated into the third foundation composition, but the amount of the silicone oil is preferably 20.0-80.0 wt.% on the basis of the entirety of the composition.

ii) A polyether-modified silicone incorporated into the third foundation composition is an organopolysiloxane graft polymer having a polyoxyalkylene group represented by the following general formula: 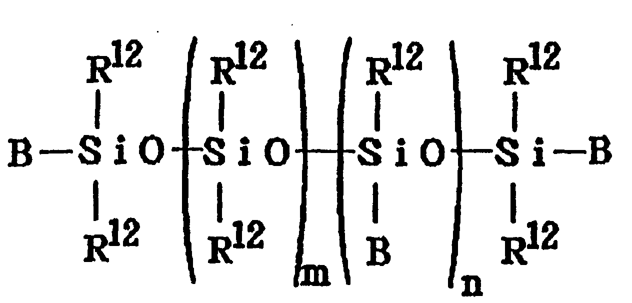 [wherein B represents a methyl group, a phenyl group, or a polyoxyalkylene group represented by the general formula: -C₃H₆O(C₂H₄O)_{b}(C₃H₆O)_{c}R¹³ (wherein R¹³ represents a hydrogen atom, an acyl group, or a C1-C4 alkyl group; and each of b and c represents an integer of 5-50); R¹² represents a methyl group or a phenyl group; m represents an integer of 50-1,000; n represents an integer of 1-40; and the molecule contains at least one polyoxyalkylene group represented by the above formula]. Specific examples of the acyl group represented by R¹³ include a formyl group, an acetyl group, a propionyl group, a butyryl group, an acryloyl group, a benzoyl group, and a toluoyl group. Specific examples of the C1-C4 alkyl group include a methyl group, an ethyl group, an i-propyl group, an n-propyl group, a t-butyl group, and an n-butyl group.

When b or c of the polyoxyalkylene group is less than 5, the polyether-modified silicone fails to exert a satisfactory viscosity-increasing effect, whereas when b or c of the polyoxyalkylene group is in excess of 50, the third foundation composition tends to induce a sticky sensation during use.

No particular limitation is imposed on the amount of the polyoxyalkylene group contained in the polyether-modified silicone, but the amount is preferably 20.0-70.0 wt.% (exclusive of 20.0 wt.%). When the amount of the polyoxyalkylene group contained in the polyether-modified silicone is 20.0 wt.% or less, the viscosity-increasing effect of the silicone is considerably lowered, whereas when the amount is in excess of 70.0 wt.%, the compatibility between the polyether-modified silicone and a silicone oil is lowered.

In the above formula, m is an integer of 50-1,000, preferably 200-600. In the above formula, n is an integer of 1-40, preferably 5-20. When m is less than 50 and n is 0, the viscosity-increasing effect of the polyether-modified silicone is not satisfactory. When m is in excess of 1,000 and n is in excess of 40, the third foundation composition tends to induce a sticky sensation during use.

No particular limitation is imposed on the molecular weight of the polyether-modified silicone and the viscosity thereof as measured at 25°C. However, in order to form a stabler gel and impart a smooth sensation to the third foundation composition, the viscosity of a 50.0 wt.% octamethyltetrasiloxane solution of the polyether-modified silicone is preferably 1,000-100,000 cst, and the molecular weight of the polyether-modified silicone is preferably 50,000-80,000.

The amount of the polyether-modified silicone incorporated into the third foundation composition is 2.0-30.0 wt.%, preferably 5.0-15.0 wt.%, on the basis of the entirety of the composition. When the amount of the silicone is less than 2.0 wt.% on the basis of the entirety of the composition, the foundation composition becomes unstable, whereas when the amount of the silicone is in excess of 30.0 wt.%, the foundation composition induces a sticky sensation during use.

iii) The amount of water incorporated into the third foundation composition is preferably 0.2-80.0 wt.%, more preferably 2.0-6.0 wt.%, on the basis of the entirety of the composition. When the amount of water is less than 0.2 wt.% or in excess of 80.0 wt.% on the basis of the entirety of the composition, the foundation composition becomes unstable.

iv) No particular limitation is imposed on the hydrophobic powder incorporated into the third foundation composition. Preferred examples of the hydrophobic powder include organic resin powders such as polyamide resin powder (nylon powder), polyethylene powder, polymethyl methacrylate powder, polystyrene powder, styrene-acrylic acid copolymer resin powder, benzoguanamine resin powder, polytetrafluoroethylene powder, and cellulose powder; silicone resin powders such as polymethylsilsesquioxane powder; and silicone rubber powders such as polydimethylsiloxane cross-linked elastomer. Hydrophobicityimparted powder; for example, dimethylsilylated silicic anhydride or trimethylsilylated silicic anhydride, may be used as hydrophobic powder. Particularly, such hydrophobicity-imparted powder and the aforementioned silicone rubber powder are preferably used in combination. The third foundation composition may contain, other than the aforementioned hydrophobic powder, a certain type of powder, and the amount of the powder preferably accounts for 50 wt.% or less of the entirety of the powder incorporated into the foundation composition. The shape of the particles of the hydrophobic powder is not particularly limited, but when the shape is spherical, the applicability of the composition can be improved.

No particular limitation is imposed on the amount of the hydrophobic powder incorporated into the third foundation composition, but the amount is preferably 1.0-60.0 wt.%, more preferably 10.0-50.0 wt.%, on the basis of the entirety of the composition. The amount of the hydrophobic powder is preferably 3-8 parts by weight on the basis of 1 part by weight of the aforementioned polyether-modified silicone.

If necessary, each of the above-described first to third foundation compositions may further contain another ingredient as an auxiliary ingredient, so long as the ingredient does not impede the intended effects of the present invention.

Examples of the auxiliary ingredient include hydrocarbon oils such as liquid paraffin, liquid isoparaffin, and squalane; fats and oils such as olive oil, palm oil, coconut oil, macadamia nut oil, and jojoba oil; higher alcohols such as isostearyl alcohol; higher fatty acids; ester oils such as isopropyl myristate; ultraviolet absorbents such as benzophenone derivatives, para-aminobenzoic acid derivatives, para-methoxycinnamic acid derivatives, and salicylic acid derivatives; humectants, blood flow stimulants; refrigerants; antiperspirants; antibacterial agents; skin activation agents; antiphlogistic agents; vitamins; antioxidants; antioxidation aids; coloring agents; preservatives; and perfumes. Such an ingredient may be incorporated, as an auxiliary ingredient, into the foundation composition.

Particularly, a coloring agent is incorporated into each of the first to third compositions in order to conceal skin blemishes bearing a color to some extent, such as skin pores (for example, as described below, each skin pore assumes a reddish or dark tone, and thus the tone of the skin pore clearly differs from that of the area surrounding the skin pore).

The aforementioned essential ingredients and, if necessary, the aforementioned auxiliary ingredient may be mixed or kneaded by use of an apparatus such as a kneader, a mill, a roller, or a mixer, to thereby produce a paste-like foundation composition having a desired viscosity.

Mixing of the aforementioned powder ingredient may be carried out by use of a conventional mixing machine. If possible, mixing of the powder ingredient is preferably carried out by use of a mixing machine having a strong shear force.

### (B) Second layer finishing composition

The second layer finishing composition contains, as an essential ingredient, "powder with minimized regular reflection and diffuse reflection" (hereinafter the powder may be referred to as "reflection-minimized powder").

The multi-layer cosmetic composition of the present invention is a makeup cosmetic composition containing the aforementioned first layer foundation composition which is applied onto the skin and the second layer finishing composition which is applied onto the first layer of the foundation composition. The multi-layer composition is used for concealing skin pores etc. completely, and for creating a natural appearance.

Skin pores are not merely present on the skin. The periphery of each skin pore is protuberant and the central portion forms a dent. [Each skin pore has a size of 0.3-1.5 mm and a depth of 0.1-0.5 mm. In addition, since each skin pore assumes a reddish or dark tone, the tone of the skin pore clearly differs from that of the surrounding area of the skin pore.]

Therefore, in order to correct skin pores and smooth the surface of the skin completely, a large amount of a paste-like material which provides a several-mm layer must be applied onto the skin. However, application of such a material onto the skin is not a realistic solution, considering that the thickness of a cosmetic layer is usually about 20 µm at most when conventional cosmetics and cosmetic methods are applied

When only the first layer foundation composition is used according to a conventional cosmetic method, making skin pores invisible to the naked eye is difficult.

When only the first layer composition of the multi-layer cosmetic composition of the present invention is applied onto the skin, the smoothness of the skin is greatly improved as compared with that of the skin onto which the composition is not applied. However, through application of only the first layer composition, satisfactory smoothness of the skin is difficult to realize. Consequently, making skin pores invisible to the naked eye is difficult. Such difficulty in concealment of skin pores is attributed to surface reflection at the boundary between air and the surface of the first layer on the skin. This is supported by the fact that a photograph in which skin pore irregularities are barely observable can be obtained by placing polarizing filters in front of a lens and a light source such that polarization planes cross each other to thereby eliminate surface reflection light.

Therefore, if the surface reflection of the first layer can be reduced sufficiently, skin pores can be made invisible to the naked eye. The multi-layer cosmetic composition of the present invention can realize "reduction in surface reflection of the first layer" by containing powder which exerts the effect of reducing surface reflection in the second layer.

Surface reflection will now be considered in more detail. Microscopically, surface reflection is an optical phenomenon caused by the difference in refractive index between air and a medium. The reflection direction is expressed according to Snell's law, and the intensity of surface reflection light is expressed by the Fresnel equations. From the macroscopic standpoint the following is noted; i.e., when the surface of the skin is observed by the naked eye, if a relatively large number of optically smooth planes parallel to the light-irradiated plane are present on the skin surface which, in general, has minute irregularities, the intensity of light reflected from the skin at the regular angle with respect to an incident angle of light applied to the skin (regular reflection light) is higher than that of light reflected from the skin at any angle, other than the regular angle, with respect the incident angle (diffuse reflection light). Consequently, shadows tend to be produced, and skin blemishes such as skin pores are clearly observable. Therefore, in order to make irregularities of skin pores invisible to the naked eye, regular reflection light must be minimized.

In the case in which powder having a flat plane on its particle surface, such as mica or sericite, is incorporated into the second layer composition of the multi-layer cosmetic composition, when the second layer composition is applied onto the skin, skin pores are clearly observable, since the flat plane of each particle is easily aligned parallel to the surface of the skin, and thus the intensity of regular reflection light becomes high. In order to minimize regular reflection light, particle surfaces must be aligned randomly with respect to the skin surface. In general, spherical powder realizes such a random alignment of the particle surfaces most readily. Therefore, when spherical powder is incorporated into the second layer composition of the multi-layer cosmetic composition, such surface reflection can be minimized (similar to the case of the conventional multi-layer cosmetic composition).

Diffuse reflection light, which is the other type of surface reflection light, will now be considered. Macroscopically, diffuse reflection light is light that is reflected at any angle, exclusive of the regular reflection angle, with respect to an incident angle. When minute planes of a light-irradiated plane are completely randomly aligned, the irradiated plane becomes a complete diffuse reflection plane, and thus the brightness of a certain region of the plane becomes constant when viewed from any angle. Therefore, when such a diffuse reflection plane is present, skin blemishes can be concealed. Therefore, it is reasonable that a light diffuse reflection function is imparted to the second layer composition which is applied onto the first layer composition, in order to conceal the irregularities created by skin pores, etc. However, in relation to the cosmetic composition, imparting a light diffuse reflection function to the second layer composition involves problems, for the reasons described below.

In relation to diffuse reflection light reflected from minute particles present on the light-irradiated plane, some of the diffuse reflection light is light reflected from a plane of each particle which is not parallel to the irradiated plane, and some of the diffuse reflection light is light which penetrates the outermost plane of each particle and is returned after repeated reflection at the boundary of particles. When light is applied to a target, if the target does not absorb the light regardless of wavelength, diffuse reflection light is recognized as white light. In the case in which a target to which light is applied is powder particles, when powder particles accumulates and the number of particle boundaries increases, the intensity of diffuse reflection light increases; i.e., whiteness becomes apparent. Such whiteness clearly reveals the presence of powder. In the case of makeup, such whiteness is regarded as a "powdery appearance," and is not preferable. In conventional multi-layer cosmetic compositions, the first layer of the composition only plays the part of adhering the second layer onto the skin, and does not exert the effect of correcting skin pores, etc. Therefore, when only the first layer composition is used, the original shape or color of skin pores, etc. cannot be concealed. Briefly, when the second layer composition is not applied onto the skin to produce diffuse reflection light, skin pores, etc. cannot be effectively concealed.

In contrast, the first layer composition of the multi-layer cosmetic composition of the present invention exerts excellent effects for smoothing and correcting skin pores, etc. Therefore, even when powder with minimized surface reflection; i.e., powder with minimized light regular reflection and light diffuse reflection, is incorporated into the second layer composition, skin pores, etc. can be concealed satisfactorily.

Reflection-minimized powder incorporated into the second layer composition of the multi-layer cosmetic composition of the present invention will next be described in more detail.

In order to meet the aforementioned conditions required for reflection-minimized powder, reflection-minimized powder particles generally assume a spherical shape. In addition, when reflection-minimized powder is applied onto the first layer composition by use of a conventional cosmetic tool such as a sponge, a brush, or a puff, the reflection-minimized powder must be applied so as to attain a minimum thickness (i.e., a thickness equivalent to one or two particles), and the first layer composition must be easily coated with the reflection-minimized powder, without causing agglomeration of particles. Briefly, refection-minimized powder is powder which typically assumes a spherical shape and which can be applied onto the skin very thinly.

Specifically, as used herein, the term "reflection-minimized powder" refers to powder satisfying the following conditions: when powder is uniformly applied onto a black gel surface, the regular reflection of the powder is suppressed to 1% or less with respect to the regular reflection of a quartz plate (hereinafter an index showing the degree of regular reflection will be referred to as "regular reflection percentage"); and when powder is uniformly applied onto the black gel surface, the diffuse reflection of the powder is suppressed to 1% or less with respect to the diffuse reflection of a standard white plate [formed from barium sulfate (BaSO₄)] (hereinafter an index showing the degree of diffuse reflection will be referred to as "diffuse reflection percentage") (the regular reflection percentage and diffuse reflection percentage will be described in more detail in Examples).

No particular limitation is imposed on the particle size, the shape, and the refractive index of the reflection-minimized powder, so long as the powder satisfies the aforementioned conditions in relation to regular reflection percentage and diffuse reflection percentage. There may be used non-treated powder formed of a variety of materials and, if necessary, surface-treated powder.

As shown in Table 1 (1), examples of the material of reflection-minimized powder particles include, but are not limited to, polymethyl methacrylate, polyethylene, nylon, silica, silicone rubber-, silicone resin (methylsiloxane polymer), silicone resin-coated silicone rubber, and polystyrene.

A yardstick of the particle size (particle shape) or refractive index of the reflection-minimized powder for satisfying the aforementioned conditions will be described below (the yardstick should be regarded as just "yardstick").

When the reflection-minimized powder assumes a spherical shape, the particle size is about 0.1-50 µm, preferably about 0.4-20 µm. When the reflection-minimized powder assumes a non-spherical shape (e.g., is amorphous), the particle size is about 0.01-10 µm.

Regardless of the shape or size of particles, the refractive index of the reflection-minimized powder is about 1.3-2.0.

A repeating structure of minute irregularities having a size one-half or less the wavelength of visible rays has the effect of reducing the effective refractive index (Mosai's principle). In order to provide such a structure on the application surface of powder, when powder particles having a size of 100-200 nm are used, the particles are agglomerated with one another, and thus the intensity of diffuse reflection light tends to increase. Powder particles having a size in excess of 50 µm may fail to have the effect of minimizing regular reflection light.

The amount of reflection-minimized powder incorporated into the second layer finishing composition is 1.0-100 wt.%, preferably 10.0-100 wt.%, on the basis of the entirety of the finishing composition. When the amount is less than 1.0 wt.% on the basis of the entirety of the finishing composition, the second layer composition has difficulty in satisfactorily concealing skin pores, etc.

In the case in which powder having a regular reflection percentage or diffuse reflection percentage of more than 1% is incorporated into the second layer finishing composition (for example, in order to increase the strength of a product form, in many cases, plate-like powder or micropowder must be incorporated), if possible, powder having a regular reflection percentage and a diffuse reflection percentage of 5% or less is preferably incorporated [as used herein, powder which has a regular reflection percentage and a diffuse reflection percentage of 5% or less and fails to satisfy the definition of reflection-minimized powder; i.e., powder having a regular reflection percentage and/or a diffuse reflection percentage of more than 1%, is referred to as "semi-reflection-minimized powder," and powder, other than reflection-minimized powder and semi-reflection-minimized powder, is referred to as "reflection powder"].

When reflection powder such as titanium oxide powder is incorporated into the second layer finishing composition, in order to realize a natural appearance, if possible, the amount of the powder incorporated into the composition is preferably reduced. Specifically, the amount of reflection powder incorporated into the second layer finishing composition is preferably less than 10.0 wt.%, more preferably 5.0 wt.% or less, on the basis of the entirety of the composition. When the amount is 10.0 wt.% or more on the basis of the entirety of the finishing composition, a powdery or white appearance attributable to the reflection powder becomes considerable, and thus the multi-layer cosmetic composition has difficulty in attaining its original purpose; i.e., imparting a natural appearance.

In summary, the amount of reflection-minimized powder incorporated into the second layer finishing composition is 1.0-100 wt.%, preferably 10.0-100 wt.%, on the basis of the entirety of the finishing composition. When non-reflection-minimized powder is incorporated into the finishing composition, if possible, semi-reflection-minimized powder is preferably incorporated into the composition (semi-reflection-minimized powder may account for 0-100 wt.% of non-reflection-minimized powder). When reflection powder is incorporated into the finishing composition, the amount of the reflection powder is preferably less than 10.0 wt.%, more preferably 5.0 wt.% or less, on the basis of the entirety of the composition.

The second layer finishing composition may contain an ingredient which can be used in a conventional makeup cosmetic composition, so long as the ingredient does not impede the intended effects of the present invention, particularly "reflection minimization effect." The second layer finishing composition may contain an oil ingredient, a surfactant, a dispersant, a plasticizer, a perfume, a preservative, a conventional powder ingredient, or a pigment ingredient.

### (C) Description of use

As described above, when the multi-layer cosmetic composition of the present invention is used, firstly the first layer foundation composition is applied onto the skin for smoothing and correcting skin blemishes, and then the second layer finishing composition is applied onto the foundation composition.

The first layer foundation composition adheres to skin blemishes such as skin pores, and smoothes and corrects the blemishes. Therefore, even when diffuse reflection powder―which powder has the effect of concealing skin blemishes through a light diffuse reflection function―is not incorporated into the second layer finishing composition, skin blemishes can be effectively concealed. When the first layer foundation composition is used, reflection-minimized powder can be incorporated into the second layer finishing composition, and thus both concealment of skin blemishes and the imparting of a natural appearance are realized.

The multi-layer cosmetic composition of the present invention can assume any product form, so long as the first layer foundation composition and the second layer finishing composition can be used simultaneously. For example, the first layer foundation composition and the second layer finishing composition may be provided in the same container. Alternatively, the multi-layer cosmetic composition may assume a "multi-layer makeup kit" in which the first layer foundation composition and the second layer finishing composition are separately provided in different containers.

### Examples

The present invention will next be described in more detail by way of Examples, which should not be construed as limiting the invention thereto. Unless otherwise specified, the incorporation amount refers to wt.% on the basis of the entirety of an incorporation target.

### Selection of reflection-minimized powder

Powder was applied onto the surface of a black silicone gel (product name: Gel·OK·Packing, product of Oba Koki) by use of a brush (Shiseido Cheek Brush 301, product of Shiseido Co., Ltd.), and was slicked such that the powder did not fall when the gel was inverted (the powder was slicked in a light incidence plane vector direction). Within one day after preparation of the gel serving as a measurement sample, the regular reflection percentage and diffuse reflection percentage of the powder were measured by use of an actinometer 10 having the below-described structure.

### <Structure of actinometer>

The sample 4 was irradiated with light from a light source 1, the light being passed though a light introduction fiber 2 and a light source lens 3. The reflected light was passed through a light-receiving lens 5 and a spectrometer introduction fiber 6, and was subjected to spectroscopic treatment in a spectrometer 7. The resultant light was detected by a detector 8, and the regular reflection percentage and diffuse reflection percentage at 546 nm were calculated by use of processing software (see Fig. 1, wherein arrows show the direction of progress of light and measurement data).

Specifically, the actinometer employed in the Example has the following structure:
Light source 1: a halogen lamp (product of OSRAM, BELLAPHOT, 12V, 30W)
Light introduction fiber 2: product of Mitsubishi Densen Kogyo, round-round alignment, Φ200 µm x 61 fibers
Light source lens 3: lens 10 mm (focal length: 60 mm)
Light-receiving lens 5: lens 10 mm (focal length: 60 mm)
Spectrometer introduction fiber 6: product of Mitsubishi Densen Kogyo, three vertical lines on the side of the spectrometer 7, round alignment on the side of the sample 4, Φ200 µm × 31 fibers
Spectrometer 7: MD-10TP (product of Japan Spectroscopic Co., Ltd.), diffraction grating (200 grooves/mm), stopper (0.25 mm), filter L-37 (390-680 nm), center wavelength (546 nm)
Detector 8: C5964-0911 (product of Hamamatsu Photonics)
Processing software: ATLAS for WINDOWS ver. 2.4

### (1) Calculation of regular reflection percentage

The surface of the measurement sample was irradiated with light at 45° with respect to the normal of the irradiated surface, the light being passed through a first polarization filter; the regular reflection light was received through a second polarization filter whose polarization plane is the same as that of the first polarization filter; and the luminous energy (lumen: hereinafter the luminous energy will be represented by lumen) of the regular reflection light was obtained. The ratio (percentage) of the luminous energy of the regular reflection light to that of reflection light of a quartz plate (thickness: 5 mm) serving as a control was calculated, and the ratio was regarded as the "regular reflection percentage."

### (2) Calculation of diffusion reflection percentage

The surface of the measurement sample was irradiated with light at 45° with respect to the normal of the irradiated surface, the light being passed through a first polarization filter; light reflected at 0° with respect to the normal was received through a second polarization filter whose polarization plane crosses that of the first polarization filter; and the luminous energy of the reflected light was obtained. The ratio (percentage) of the luminous energy of the reflected light to that of reflection light of a white standard plate formed of barium sulfate (BaSO₄) (product of Hitachi Ltd.) serving as a control was calculated, and the ratio was regarded as the "diffuse reflection percentage."

The results are shown in Table 1 [Table 1 (1) shows reflection-minimized powders, Table 1 (2) shows semi-reflection-minimized powders, and Table 1 (3) shows reflection powders].

**Table 1 (1)**

| Powder | % Diffuse reflection | % Regular reflection |
|---|---|---|
| Methylsiloxane copolymer | | |
| (particle size: 4.5 µm, refractive index: | 0.48 | 0.34 |
| 1.4, particle shape: spherical) | | |
| Methylsiloxane copolymer | | |
| (particle size: 12 µm, refractive index: | 0.39 | 0.5 |
| 1.4, particle shape: spherical) | | |
| Polyethylene | | |
| (particle size: 10 µm, refractive index: | 0.79 | 0.79 |
| 1.52, particle shape: spherical) | | |
| Nylon | | |
| (particle size: 4.0 µm, refractive index: | 0.92 | 0.66 |
| 1.53, particle shape: spherical) | | |
| Polymethyl methacrylate | | |
| (particle size: 10 µm, refractive index: | 0.28 | 0.47 |
| 1.49, particle shape: spherical) | | |
| Silica | | |
| (particle size: 5.0 µm, refractive index: | 0.2 | 0.33 |
| 1.46, particle shape: spherical) | | |
| Silicone resin-coated rubber powder | | |
| (particle size: 5.0 µm, refractive index: | 0.51 | 0.34 |
| 1.4, particle shape: spherical) | | |
| Polystyrene | | |
| (particle size: 6.6 µm, refractive index: | 0.66 | 0.44 |
| 1.6, particle shape: spherical) | | |
| Silica (micropowder) | | |
| (particle size: 0.016 µm, refractive | 0.94 | 0.64 |
| index: 1.46, particle shape: amorphous) | | |

**Table 1 (2)**

| Powder | % Diffuse reflection | % Regular reflection |
|---|---|---|
| Methylsiloxane copolymer | | |
| (particle size: 0.5 µm, refractive index: | 2.64 | 1.0 |
| 1.4, particle shape: spherical) | | |
| Zinc oxide (micropowder) | | |
| (particle size: 0.04 µm, refractive | 2.34 | 1.85 |
| index: 2.0, particle shape: amorphous) | | |
| Polyethylene | | |
| (particle size: 9.3 µm, refractive index: | 0.79 | 0.79 |
| 1.5, particle shape: amorphous) | | |
| Corn starch | | |
| (particle size: 10.2 µm, refractive | 2.04 | 0.87 |
| index: 1.5, particle shape: polygonal) | | |
| Allophane | | |
| (particle size: 5.0 µm, refractive index: | 1.03 | 0.86 |
| 1.6, particle shape: spherical (hollow)) | | |
| Vinylidene chloride | | |
| (refractive index: 1.6, particle shape: | 3.15 | 1.5 |
| spherical (hollow)) | | |
| Silica | | |
| (particle size: 10 µm, refractive index: | 3.34 | 1.4 |
| 1.5, particle shape: spherical (hollow)) | | |
| Silica | | |
| (particle size: 10 µm, refractive index: | 1.06 | 1.22 |
| 1.5, particle shape: plate-like) | | |
| Talc | | |
| (particle size: 5 µm, refractive index: | 2.16 | 3.45 |
| 1.57, particle shape: plate-like) | | |
| Barium sulfate | | |
| (particle size: 0.7 µm, refractive index: | 1.93 | 0.49 |
| 1.64, particle shape: agglomeration) | | |
| Kaolin | | |
| (particle size: 1.4 µm, refractive index: | 3.61 | 0.61 |
| 1.56, particle shape: plate-like) | | |

**Table 1 (3)**

| Powder | % Diffuse reflection | % Regular reflection |
|---|---|---|
| Calcium phosphate | | |
| (particle size: 20 µm, refractive index: | 6.45 | 1.05 |
| 1.59, particle shape: agglomeration) | | |
| Cellulose | | |
| (particle size: 15 µm, refractive index: | 6.19 | 1.1 |
| 1.5, particle shape: spherical) | | |
| Mica | | |
| (particle size: 2.6 µm, refractive index: | 0.4 | 11.39 |
| 1.52, particle shape: plate-like) | | |
| Titanium oxide (rutile-type) | | |
| (particle size: 0.4 µm, refractive index: | 28.66 | 8.72 |
| 2.7, particle shape: agglomeration) | | |
| Titanium oxide (micropowder) | | |
| (particle size: 0.03 µm, refractive index: | 2.67 | 5.24 |
| 1.6, particle shape: agglomeration) | | |
| Sericite | | |
| (particle size: 3.9 µm, refractive index: | 5.8 | 0.5 |
| 1.57, particle shape: plate-like) | | |
| Zinc oxide | | |
| (particle size: 0.5 µm, refractive index: | 3.4 | 10.21 |
| 2.0, particle shape: agglomeration) | | |
| Barium sulfate | | |
| (particle size: 5.0 µm, refractive index: | 6.92 | 0.34 |
| 1.64, particle shape: plate-like) | | |

The results show a specific yardstick for reflection-minimized powder, semi-reflection-minimized powder, or reflection powder.

### Evaluation of the multi-layer cosmetic composition of the present invention

Twenty panelists (women who have conspicuous skin irregularities, such as skin pores or acne scars, on their faces) enrolled in the test. After a lotion and an emulsion were applied onto the face of each panelist, the first layer foundation composition was applied onto the entirety of the face with a finger, and then the second layer finishing composition was applied onto the entirety of the face by use of a puff. Thereafter, the degree of concealment of the irregularities on the face and how natural it appeared were visually evaluated by five experts.

### (1) Evaluation items and evaluation ratings

### 1. Evaluation of concealment of irregularities

### <Evaluation ratings>

AA: the irregularities are very effectively concealed and the presence thereof is not observed.

BB: the irregularities are effectively concealed and the presence thereof is barely observed.

CC: the irregularities are slightly concealed and the presence thereof is difficult to observe.

DD: the irregularities are not concealed and the presence thereof is clearly observed.

### 2. Evaluation of how natural it appeared (the degree of a powdery appearance)

### <Evaluation ratings>

AA: a powdery appearance is not imparted; i.e., a very natural appearance

BB: a powdery appearance is barely imparted; i.e., a natural appearance

CC: a powdery appearance is slightly imparted; i.e., a slightly unnatural appearance

DD: a powdery appearance is imparted; i.e., an unnatural appearance

### (2) Formulations of the first layer foundation

### composition

Formulations according to the present invention (i.e., Examples marked with L) are shown in Table 2.

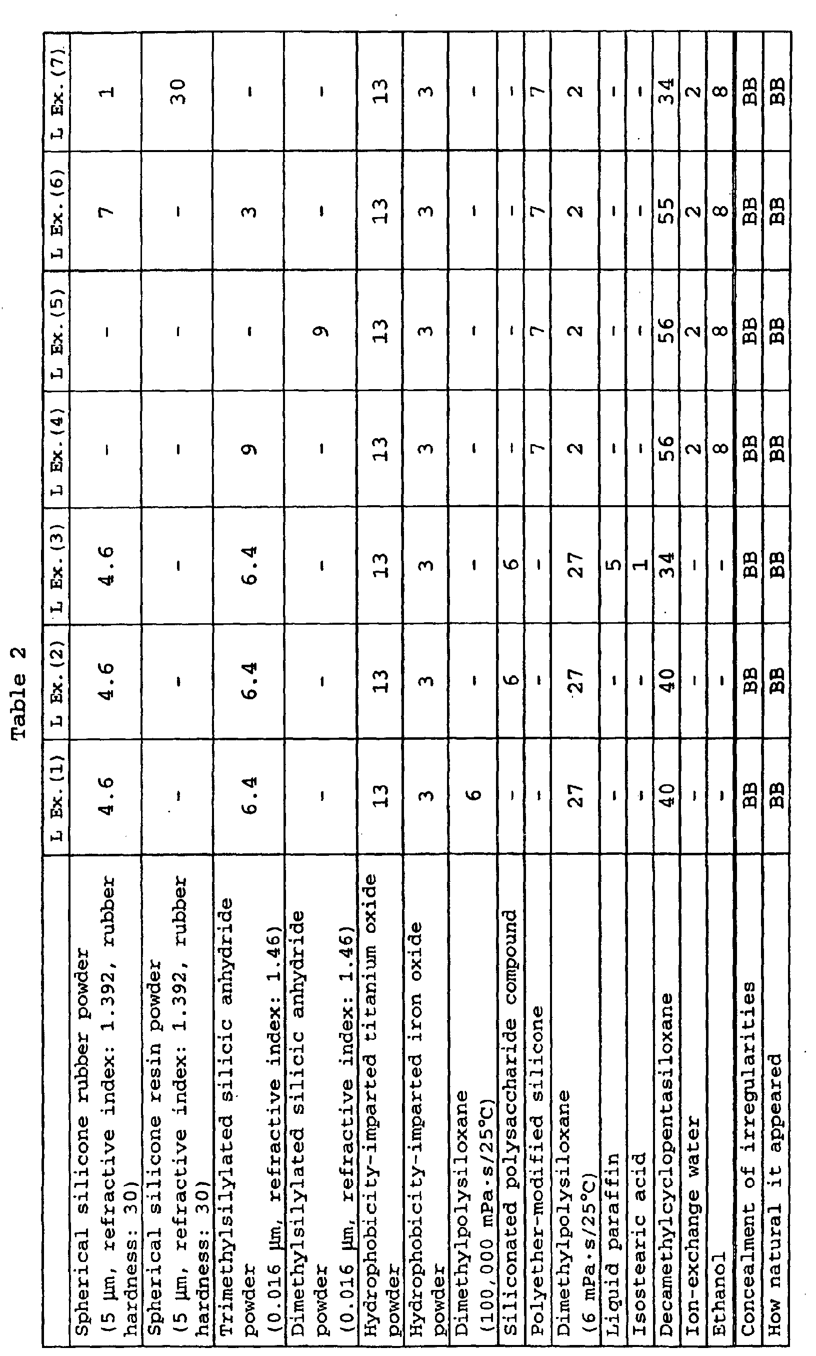

Comparative formulations (i.e., Comparative Examples marked with L) are shown in Table 3.

**Table 3**

| | L Comp. Ex. (1) | L Comp. Ex. (2) |
|---|---|---|
| Ethanol | 10 | 0 |
| 1,3-Butylene glycol | 3 | 0 |
| Ion-exchange water | 36.8 | 0 |
| Methylparaben | 0.2 | 0 |
| Acrylic copolymer | 50 | 0 |
| Volatile silicone oil (1 cs) | 0 | 50 |
| Dimethylpolysiloxane (20 cs) | 0 | 2 |
| Three-dimensional network silicone | 0 | 48 |
| Total | 100 | 100 |

When only the first layer foundation composition of the present invention was applied onto the face, the irregularities were concealed to some extent (see Table 5). In contrast, when only the comparative first layer composition was applied onto the face, the irregularities were not concealed, and the appearance in relation to the irregularities was the same as that before application of the comparative composition.

### (3) Formulations of the second layer finishing composition

Formulations according to the present invention (i.e., Examples marked with P) and comparative formulations (i.e., Comparative Examples marked with P) are shown in Table 4.

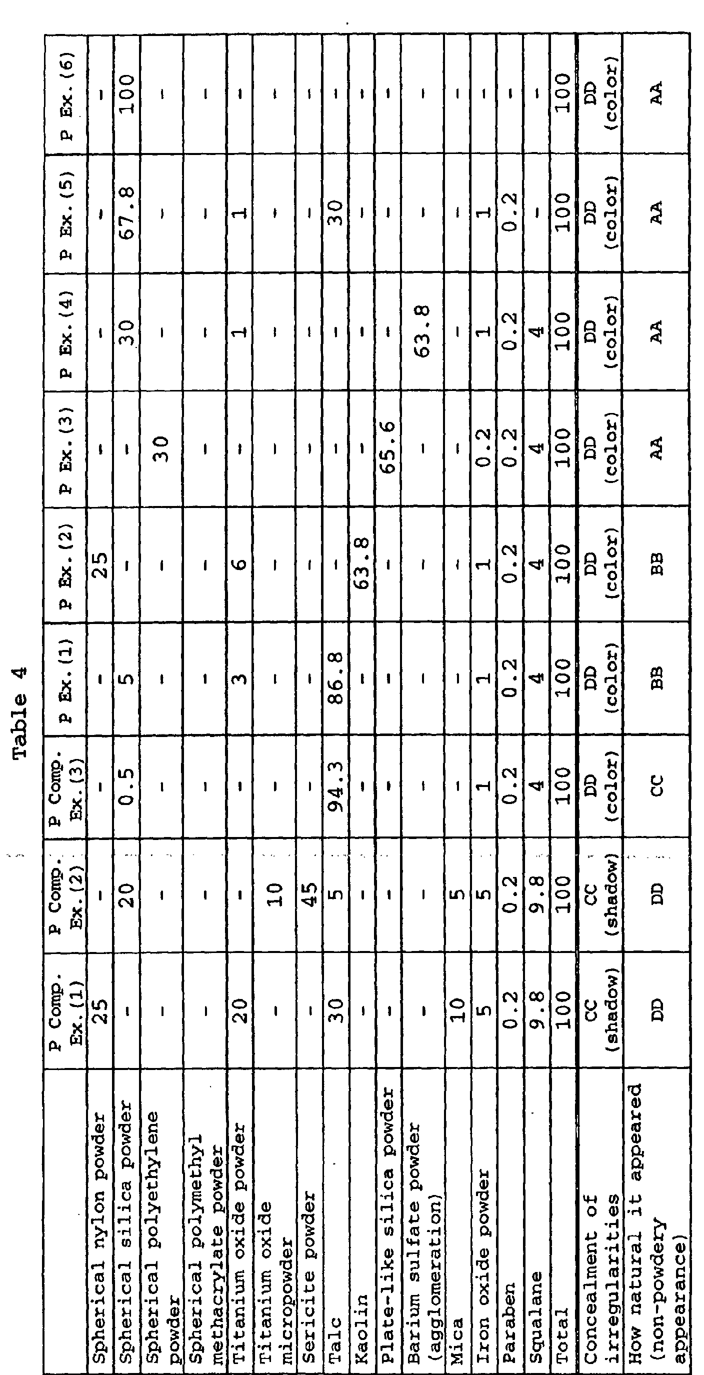

The powders shown in Table 4 include (1) spherical nylon powder (particle size: 4.0 µm, refractive index: 1.53); (2) spherical silica powder (particle size: 5.0 µm, refractive index: 1.46); (3) spherical polyethylene powder (particle size: 10 µm, refractive index: 1.52); (4) spherical polymethyl methacrylate powder (particle size: 10 µm, refractive index: 1.49); (5) titanium oxide powder (rutile-type) (particle size: 0.4 µm, particle shape: agglomeration, refractive index: 2.7); and (6) titanium oxide micropowder (particle size: 0.03 µm, particle shape: agglomeration, refractive index: 1.6).

In order to evaluate the second layer finishing composition (the results are shown in Table 4), a conventional cosmetic foundation composition [ingredients: trimethylsiloxysilicate (10 wt.%), decamethylcyclopentasiloxane (80 wt.%), and dimethylpolysiloxane (6 mPa·s) (10 wt.%)] was applied onto the skin, and then the second layer finishing composition was applied thereon.

In the case of the comparative composition, the irregularities were concealed to some extent through diffuse reflection, but a white appearance (i.e., unnatural appearance) was imparted. In contrast, in the case of the composition according to the present invention, although a natural appearance was imparted because of minimized regular reflection and diffuse reflection, the irregularities were not concealed. (In the evaluation-related row in Table 4, the term "color" refers to the case in which the irregularities looked to assume color, and the term "shadow" refers to the case in which the irregularities were seen as shadows. Hereinafter the same meanings apply for the same languages.

### (4) Effects of combination of the first layer foundation composition and the second layer finishing composition

Effects of combination of the first layer foundation composition and the second layer finishing composition (i.e., the first layer foundation composition is applied onto the skin, and then the second layer finishing composition is applied thereon) were evaluated. (For comparison, the first layer foundation composition or the second layer finishing composition is used singly). Specific combinations of these compositions and the results are shown in Table 5 (in Table 5, symbol * refers to the first layer foundation composition or the second layer finishing composition used in each combination).

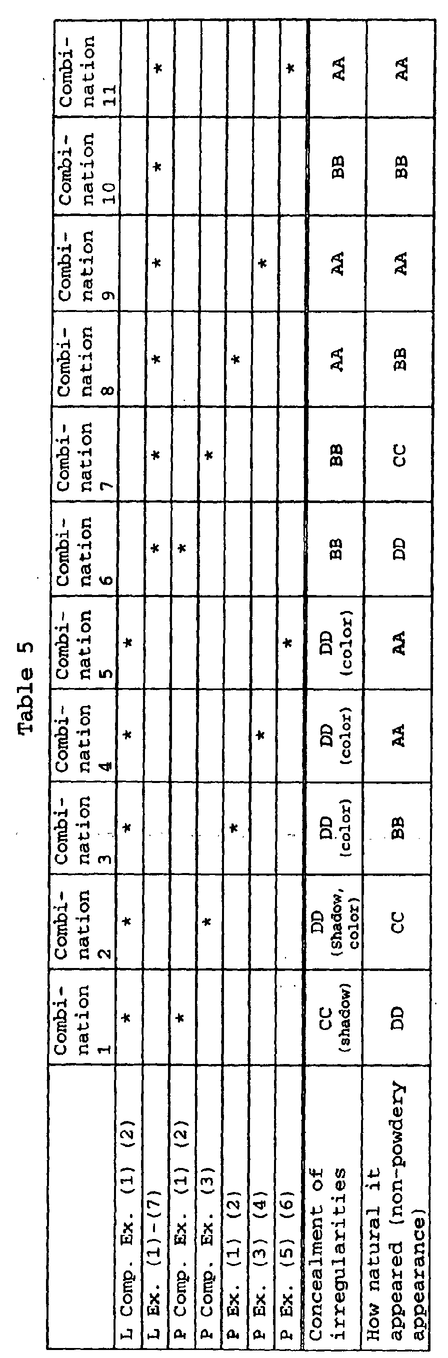

### (4) Results

Table 5 shows the following: (1) when only the first layer foundation composition of the present invention is used (Combination 10), the effect of concealment of the irregularities is moderate and not remarkable; (2) when the comparative first layer foundation composition is used (Combinations 1 through 5), the effect of concealment of the irregularities is clearly poor; (3) when the first layer foundation composition of the present invention and the comparative second layer finishing composition are used in combination (Combinations 6 and 7), the effect of concealment of the irregularities is obtained, but a powdery appearance is imparted; i.e., finishing of makeup tends to become unnatural; and (4) when the first layer foundation composition of the present invention and the second layer finishing composition of the present invention are used in combination (Combinations 8, 9, and 11), excellent results are obtained in terms of both the effect of concealment of the irregularities and a natural appearance.

The above results show that the multi-layer cosmetic compositions of the present invention have excellent utility with respect to skin blemishes, as compared with conventional cosmetic compositions.

### Industrial Applicability

The present invention provides a makeup cosmetic composition which conceals skin pores, etc., and provides a natural appearance when "powder with minimized regular reflection and diffuse reflection" is used.

## Claims

1. A two-layer makeup cosmetic composition comprising a first layer foundation composition and a second layer finishing composition, wherein the first layer foundation composition and the second layer finishing composition are as follows:
the first layer foundation composition: (1) a composition containing a silicone oil having a viscosity of 15,000,000 cps or less as measured at 25°C and a powder ingredient; (2) a composition containing a siliconated polysaccharide compound, and a silicone oil having a low viscosity and/or a powder ingredient; or (3) a composition containing i) a silicone oil, ii) a polyether-modified silicone represented by the following formula, iii) water, and iv) hydrophobic powder: 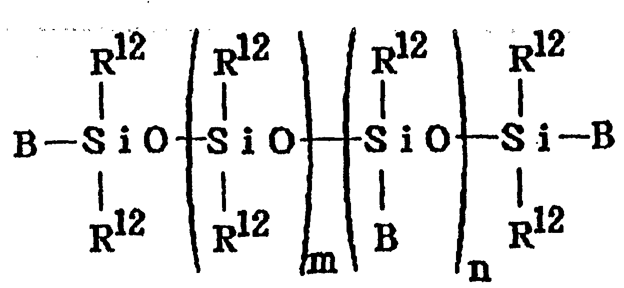 [wherein B represents'a methyl group, a phenyl group, or a polyoxyalkylene group represented by the formula -C₃H₆O(C₂H₄O)_{b}(C₃H₆O)_{c}R¹³ (wherein R¹³ represents a hydrogen atom, an acyl group, or a C1-C4 alkyl group; and each of b and c represents an integer of 5-50); R¹² represents a methyl group or a phenyl group; m represents an integer of 50-1,000; n represents an integer of 1-40; and the molecule contains at least one polyoxyalkylene group represented by the above formula]; and
the second layer finishing composition: a composition containing powder with minimized regular reflection and diffuse reflection in an amount of 1-100 wt.% on the basis of the entirety of the finishing composition.

2. The two-layer makeup cosmetic composition according to claim 1, wherein the amount of the powder with minimized regular reflection and diffuse reflection incorporated into the second layer finishing composition is 10-100 wt.% on the basis of the entirety of the finishing composition.

3. The two-layer makeup cosmetic composition according to claim 1 or 2, wherein the powder with minimized regular reflection and diffuse reflection has a regular reflection percentage of 1% or less and a diffuse reflection percentage of 1% or less.
